# EUROPEAN PATENT SPECIFICATION

(11) **EP 1 005 311 B1**
(45) Date of publication and mention of the grant of the patent: **03.07.2002**
(21) Application number: 98907318.4
(22) Date of filing: 25.02.1998
(51) Int. Cl.: A61F 13/15

(54) **AN ABSORBENT ARTICLE THAT INCLUDES A LIQUID BARRIER WITH IMPROVED SEALING**
ABSORBIERENDER ARTIKEL MIT EINER FLÜSSIGKEITSBARRIERE MIT VERBESSERTER DICHTHEIT
ARTICLE ABSORBANT COMPORTANT UNE BARRIERE POUR LIQUIDE DOTEE D'UNE ETANCHEITE AMELIOREE

(30) Priority: 26.02.1997 SE 9700693
(43) Date of publication of application: 07.06.2000
(73) Proprietor: SCA Hygiene Products AB, 405 03 Göteborg (SE)
(72) Inventor: SIMMONS, Eva, S-431 66 Mölndal (SE); RÖNNBERG, Peter, S-431 33 Mölndal (SE); GUSTAFSSON, Anders, S-427 35 Billdal (SE)
(74) Representative: Larsson, Karin
(86) International application number: SE9800341
(87) International publication number: WO9837843

(56) References cited:
- EP-A- 0 534 488
- US-A- 5 445 627

## Description

The present invention relates to absorbent articles, such as diapers or incontinence guards, that provide a better sealing effect against the wearer's skin than earlier known articles of this kind and that includes an absorbent body disposed between a liquid-impermeable bottom sheet which is intended to lie distal from the wearer in use, a liquid-permeable upper sheet which is intended to lie proximal to the wearer, and either 1) at least one longitudinally extending liquid barrier on each side of the centre line of said upper sheet, made of essentially liquid-impervious material and fastened along or adjacent to a respective longitudinally extending side extremity of the article and comprising a free elastic sealing edge intended to be stretched against the wearer, or 2) above the upper sheet, a liquid-impermeable top sheet which is intended to lie against the wearer, includes elastic for shaping the article to the wearer's body, and includes apertures intended to lie in register with the anus and the urethra orifice of the wearer, around which apertures elastically puckered sealing edges are disposed in the top sheet. The invention also relates to a method of achieving, in such articles, an improved sealing ability against the skin of an intended wearer, at a given available elongation, by at least one sealing edge on each side of the centre line.

The first type of absorbent article to which the invention relates will include a liquid-impermeable sheet which is intended to lie distal from the wearer in use, an absorbent body, and an upper liquid-permeable sheet which lies proximal to the wearer in use. When the absorbent article concerned is a diaper or an incontinence guard, the article will also include flexible side flaps or wings that extend laterally beyond the absorbent body on both sides thereof and elastic devices that extend longitudinally along the free side extremities of the side-flaps at least within that part which is intended to form the crotch part of the article in use, said flaps and elastic devices enabling the absorbent article to be fitted to the wearer. These elastic devices function as leg elastic when the article is worn. Thus, when the article is donned, the elastic elements will be stretched and hold the side flaps tightly against the wearer.

Present-day absorbent articles have a very high absorbency and can also retain liquid under pressure. The greatest problems occur in the event of rapid liquid discharges. In such cases, it is necessary for a large volume of liquid to pass first through the top liquid-permeable sheet and then be absorbed by and dispersed in the absorbent body. This does not take place instantaneously. The time taken for the liquid to pass through the top sheet may be from about one to two minutes, during which time liquid will run out to the extremities of the article and leak therefrom. These problems are addressed with the aid of so-called liquid barriers or inner cuffs or side-flaps that are intended to resist liquid leakage in the event of rapid liquid discharges. The originally used cuffs were intended primarily to retain faeces and were formed by folding a part of the liquid-permeable top sheet around an elastic thread. In recent times, manufacturers have begun to produce the cuffs from a liquid-impervious material so as to also retain liquid.

When the leg elastic on the absorbent article is stretched and the article fastened on the wearer, the elastic element of the liquid barriers will also be stretched, thereby raising the barriers up. The elastic element of the barriers will hold the barrier edges under tension against the wearer. The upstanding liquid barriers then form beneath the wearer a "trough" in which a rapidly discharged large volume of urine can be accommodated during the time required for the liquid to pass through the upper liquid-permeable sheet. Attempts to improve the effectiveness of such liquid barriers have hitherto been directed towards the use of denser materials and towards flaps of sufficiently large size.

One drawback, however, is that liquid will rise above the brim of the barriers and leak out when the volume of liquid discharged is excessive or when the wearer sits or lies so as to press together the space between the upstanding liquid barriers.

Thus, current absorbent articles, such as diapers or incontinence guards, may include along the outer longitudinal extremities of the article sealing edges that are intended to lie tightly around the wearer's thighs and shape the article to the wearer's body, as well as a pair of inner cuffs or liquid-barriers which lie inwardly of the outer longitudinal extremities and which are intended to form an impervious barrier against rapidly discharged liquid that is not absorbed immediately by the absorbent body of said article. These inner cuffs shall thus be capable of withstanding a relatively high liquid pressure over a limited period of time of the order of a minute or so. The liquid has been absorbed by the absorbent body when this time period has lapsed. Also available are articles which include transverse cuffs that seal-off the transverse extremities of the absorbent body.

Absorbent articles which lack the aforedescribed inner liquid barriers are also available. In such cases, the outer longitudinal sealing edges form the sole liquid barriers of the absorbent body.

Also available are absorbent articles of the second type to which the invention relates and which include a top liquid-impermeable sheet that is intended to lie proximal to the wearer in use and that includes elastic threads for shaping the article to the wearer's body. This top liquid-impermeable sheet includes at least one aperture for register with the wearer's anus and urethra orifices. Elastic is provided around at least a part of the circumference of the aperture or apertures so as to shape the edges of the aperture to the wearer and to form a seal. Situated beneath the top liquid-impermeable sheet is an absorbent body which is enclosed between an upper liquid-permeable sheet and a bottom liquid-impermeable sheet, such that the absorbent body will hang down beneath the wearer with the bottom liquid-impermeable sheet lying distal therefrom.

The inner cuffs are comprised partly of a thin barrier sheet of essentially liquid-impervious inelastic material, e.g. nonwoven, and partly an elastic device which puckers that edge of the liquid barrier which lies against the wearer. The unresilient and inelastic material is fastened along one longitudinal extremity either to the upper liquid-permeable sheet of the absorbent article so that no liquid can pass between the two sheets, or to the bottom liquid-impervious sheet along a side extremity of the article. The elastic device is fastened along the other edge of the unresilient material, so as to gather the liquid barrier together and therewith form a puckered edge, which will be extended partially when the article is donned. Puckering of this edge is normally achieved with an elastic thread placed in a channel in the inelastic material, said channel being formed by folding over and welding one edge of the material. When the absorbent article is donned, the puckered edge will be stretched partially, the extent of this stretch being dependent on the size of the wearer and how the article is donned.

The described liquid barriers or leg elastic cannot be used in absorbent articles held against the body of the wearer by special elastic pants or panties. These absorbent articles may comprise other sealing edges which are not stretched and held tensioned against the body. Thus, EP-A1-0 534 488 describes sealing gaskets which are preformed to extend outward from the central part of an absorbent article, which is illustrated by a sanitary napkin in the description of the preferred embodiment. The gaskets may be formed by looping a strip of material, such as a non-woven material, so as to form a compliant cuff which bears against the user's body in a comfortable manner. In one embodiment the gaskets are attached directly to the extremities of the absorbent article, one edge of the gasket strip being attached to the upper sheet of the absorbent article and the other edge to the lower sheet. The looping of the material form cavities for imparting compliancy and stability to the gaskets and the looping may also enclose elastic elements that are placed in tension when applied to the article so as to impart an arcuate shape to the article.

In another embodiment the gasket material is an elastic material which is attached to the longitudinally extremities of the sanitary napkin. The material is placed in tension when applied to the sanitary napkin. The purpose of doing so is to impart an arcuate shape to the article.

The sanitary napkin is placed in a panty crotch during use and is pressed against the wearer by the force from the panty. Thus, the force applied from the sanitary napkin against the wearer is indirectly caused by pressure from the panty. The gasket embodiments including elastic elements do not cause the force against the body of the wearer. The purpose of the elastic elements are, if they are used, to impart an arcuate shape. This differs from the diaper or incontinence guard according to the invention with a barrier element including an elastic device, wherein the barrier element will get stretched against the wearer during use and thereby creating a liquid barrier with a good sealing effect. It is the body of the user which makes the elastic device in the barrier to get tensioned.

Another type of sealants are described in US-A-5 445 627. A sanitary napkin is provided with a pair of elastically stretchable flaps adjacent transversely opposite side extremities. The flaps are in the form of straps and rising from a backsheet. Adhesives are applied on the top surface of said flaps so that the flaps may be adhesively fastened to the user's skin. The intention is to compensate for a shift of the basic body of the napkin relative to the wearer's body. Thus, the napkin is made to adhesively adhere to the user's skin. This is not a case where an elastic barrier element is stretched against the wearer to obtain a good sealing effect.

The object of the present invention is to provide a method of improving the ability of an absorbent article comprising leg elastic to remain tight against a wearer, with the aid of various measures. Another object of the invention is to provide an absorbent article comprising leg elastic and having improved sealing properties with respect to the wearer of the article.

The invention will now be described with reference to the accompanying drawings, in which
Figs. 1a, b, c illustrate measuring equipment used to determine the leakage pressure or breakthrough pressure for an elastic barrier material. Fig 1a shows the equipment without any applied material, Fig 1b shows the equipment with material applied in a stretched state, and Fig. 1c is a principle illustration of how the leakage pressure is determined;
Figs. 2a, b illustrate schematically a pore in a liquid barrier and the principle of determining the weighted mean value cos θm and determining the radius r;
Fig. 3a illustrates a conventional diaper or incontinence guard with upstanding liquid barriers;
Figs. 3b, c are enlarged principle views of a section through the region B in Fig. 3a, firstly with respect to a conventional diaper (3b) and secondly for an embodiment of the invention (3c);
Figs. 4a, b illustrate the principle of calculating the available elongation or stretch;
Fig. 5a is a graphic illustration showing the measured breakthrough pressures for three different liquid barriers;
Fig. 5b is a comparison diagram illustrating calculated and measured breakthrough pressures for the best liquid barrier in Fig. 5a, at different available degrees of elongation;
Fig. 5c is a diagrammatic illustration of lowest breakthrough pressures for liquid barriers constructed in accordance with the invention;
Fig. 6a is a graphic illustration of the measured breakthrough pressures for a conventional upstanding liquid barrier on the one hand and for two embodiments of the invention on the other hand;
Fig. 6b is a comparison diagram illustrating calculated and measured breakthrough pressures at different available elongations for one of the inventive embodiments shown in Fig. 6a;
Fig. 7 illustrates the measured breakthrough pressures for a conventional upstanding liquid barrier on the one hand arid for a further embodiment of the invention on the other hand;

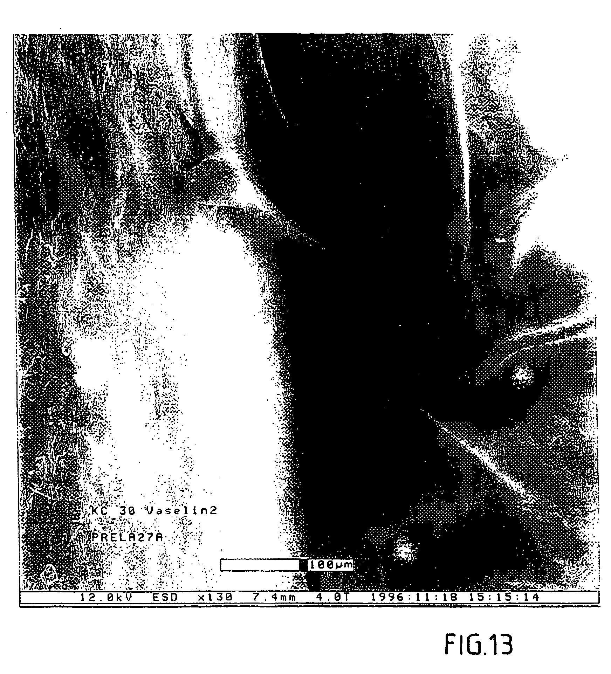

Figs. 8-14 are reproductions of photographs taken with an electron microscope of different liquid barriers at different available elongations; and
Figs. 8a-12a are views corresponding to the photograph reproductions in Figs. 8-12.

An absorbent article, such as a diaper, is manufactured for use by persons of different sizes. This is achieved by gathering together, or puckering, liquid barriers and side extremities with the aid of elastic. These liquid barriers and side extremities will stretch to different extents in accordance with the size of the wearer, and the tension around the edge of the barrier will thus vary in dependence on the size of the wearer.

Tension in the barrier elastic can be expected to have significance in studies on the sealing property of a liquid barrier, and consequently the extent to which the barrier is stretched will also be significant. A significantly stretched barrier will exert significant tension against the wearer's skin and can be expected to provide a better seal than corresponding barriers that are stretched to a lesser extent. One wish in this respect has been to produce the highest possible tension in the liquid barrier and therewith obtain the best possible sealing effect. However, it is not possible to use elastic in which the tension is excessively high, since the absorbent article will then be uncomfortable to wear and leave marks in the skin.

The term "available elongation or stretch" can be used when considering the extent to which a liquid barrier is stretched.

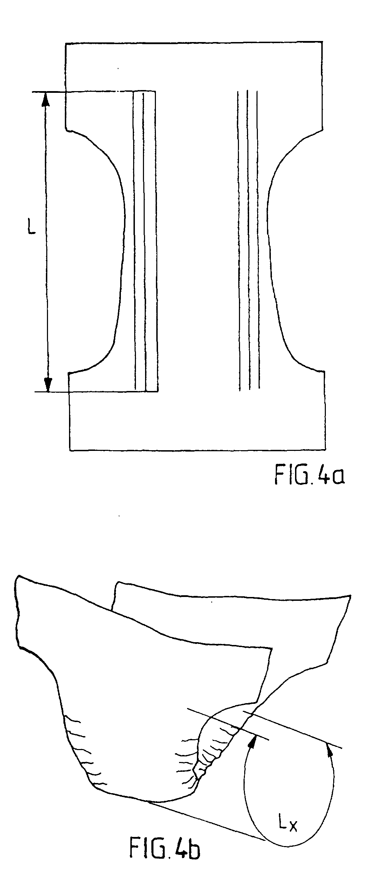

In the manufacture of the absorbent article, e.g. a diaper, the elastic material, which has a given degree of stretchability, is "locked" firmly to remaining non-stretchable material, normally nonwoven. The extent to which the elastic material is stretched in the manufacture of the article cannot be exceeded when the article is in use, since the elastic material is firmly secured to a non-stretchable material. This is shown in Fig. 4a. The elastic material has the length **L** at this point.

The non-stretchable material to which the elastic material is locked is puckered somewhat when the diaper is placed on the wearer's body. The elastic material has then contracted to the smaller length **Lx.**

The available stretch or elongation **X** is the extent to which the material can be stretched from the user state to the maximum stretched state of the product. This can be expressed by the formula: **L** = **Lx** ((**X**/100) + 1), where **X** is the available stretch or elongation in percent.

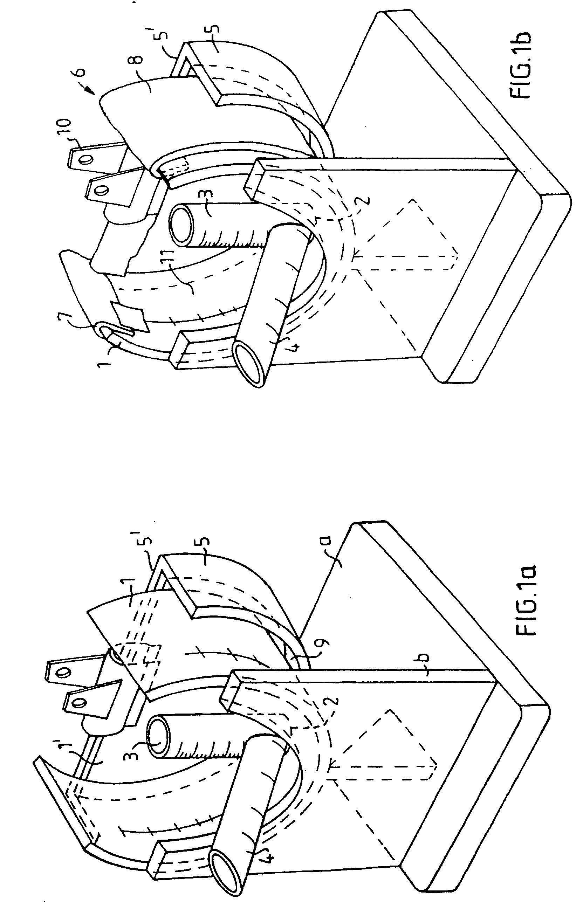

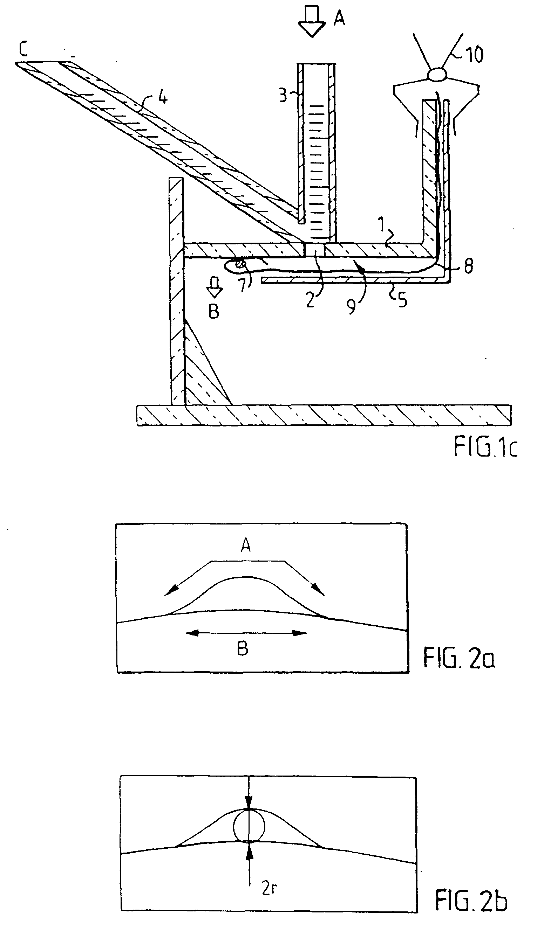

Test equipment was constructed with the intention of studying the sealing effect achieved between a liquid barrier or some other puckered barrier and the wearer's skin. This equipment is shown in Figs. 1a, 1b and 1c and comprises a Plexiglas stand which includes a base plate **a** and an upstanding support plate **b**. A first upwardly open, semi-cylindrical element 1 is fastened horizontally to the upstanding support plate **b** and has around its periphery a scale which denotes the available elongation or stretch. One end of the semi-cylindrical element is attached to the support plate while the other end has an end-wall 1'. Provided at the very bottom of the semi-cylindrical element 1 is a hole 2 to which a vertically upstanding filling tube 3 and an inclined measuring tube 4 lead, both of said tubes having a scale expressed in mm water. The equipment also includes a loose second semi-cylindrical element 5 whose diameter is somewhat larger than the diameter of the first semi-cylindrical element 1 and which has one side open and an end-wall 5' at its other end.

As shown in Fig. 1b, a measuring operation is carried out by securing a liquid barrier around the outer periphery of the first semi-cylindrical element and fastening said barrier around the upper edges. The elastic part 7 is directed towards the attachment of the semi-cylindrical element to the support plate b, and the liquid barrier material is folded around the end-wall 1' of the first semi-cylindrical element 1 on the other side. The elastic part is fastened along the scale on the semi-cylindrical element so as to enable the available elongation or stretch to be read-off. The end-wall 5' of the second semi-cylindrical element 5 is placed against the end-wall 1' of the first semi-cylindrical element with said upfolded part of said barrier material located therebetween and pressed thereagainst with the aid of a clamp 10, such as to obtain a small clearance 9 between the cylindrical walls. Synthetic urine is introduced through the vertical tube 3. The liquid barrier is first weighted down so as to fill the clearance between the semi-cylindrical elements. A liquid pressure is thereafter built-up against the elastic edge 7 at the same time as a liquid column is formed in the tubes 3, 4, where the pressure can be read-off. Liquid is introduced until leakage occurs at arrow B (Fig. 1c) at the breakthrough pressure.

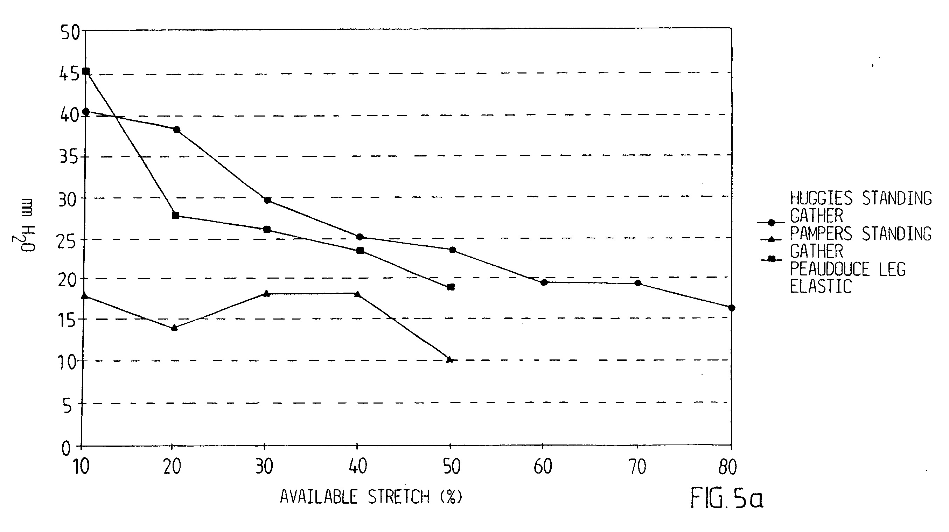

Three available types of liquid barriers, Huggies standing gather, Pampers standing gather and Peaudouce leg elastic, have been studied with this equipment, the leakage tendency with the elastic element stretched to and locked at different available elongations being measured. The liquid pressure at which leakage will occur in respect of a barrier stretched to a given extent, i.e. a barrier that has a given available elongation or stretch, has been determined with the aid of the test equipment, this pressure being found to vary in dependence on the extent to which the puckered edge is stretched. The measured values are shown in the diagram in Fig. 5a. As will be evident from the Figure, however, different barriers give different breakthrough pressures at the same available stretch or elongation. It thus appears that the sealing effect is influenced by other factors than solely the tension in the elastic material.

The invention is based on an attempt to provide an improved sealing effect on the basis of factors other than the actual tension in the elastic.

On the basis of the theory that leakage does not occur merely because the elastic in the barrier material releases its contact with the wearer's skin but first occurs through the through-penetrating pores or channels that are formed between the wearer's skin and the folds in the puckered edge of the barrier material, endeavours have been made to create a model from which the leakage pressure can be determined theoretically and thereby become aware of those parameters that shall be influenced in order to achieve a better sealing effect.

The capillary pressure of the pores in porous structures can be calculated with the Laplace equation.

According to Laplace, the capillary pressure ΔP = 2γ cosθ/r, where γ is the surface tension of the liquid, θ is the wetting angle of the liquid to the material in the capillary walls, and r is the radius of the capillary. When θ is greater than 90°, cosθ is negative and ΔP is consequently also negative. The capillary wall is hydrophobic and the resultant pressure ΔP can be said to describe the breakthrough pressure, i.e. the maximum pressure a capillary or pore can withstand. When θ is less than 90°, the capillary wall is hydrophilic and ΔP and cosθ are positive. Liquid is then "sucked" into the pores.

When studying the pressure in a capillary or pore, where the wall consists of several materials, such as in a pore formed between skin and a fold in a liquid barrier, the proportion of circumference of each material must be weighed together so as to provide a mean value of cosθ, hereinafter designated cosθm. The breakthrough pressure will then be ΔP = 2γ cosθm/r.

In the present case, the walls of the pores consist partly of an hydrophilic material, i.e. skin, which has a wetting angle of less than 90°, and partly of the hydrophobic material in the liquid barrier, which has a wetting angle above 90°.

Cosθm is the weighted mean value of the pore wall's cosθ-values and is calculated in the manner illustrated in Fig. 2a, where A designates the circumference proportion hydrophobic wall and B designates the circumference proportion hydrophilic wall, where
A + B = 1. Cosθm will therewith equal A·cosθ_{fob} + B·cosθ_{fil}.

As described below, trials have been carried out with the intention of checking whether or not the described model can be used as a basis on which the breakthrough pressure can be evaluated.

The wetting angle of the skin varies in accordance with the state of the skin, i.e. whether the skin is clean or dirty, for instance. Measuring equipment comprised of Plexiglas with a wetting angle of 77°, which lies close to the mean value of the wetting angle of the skin (about 74°), was used for comparison purposes. Measurements were carried out on the commercial liquid barrier that produced the best sealing result according to Fig. 5a, i.e. Huggies standing gather which has a wetting angle of 120°. The liquid used was synthetic urine. γ is the surface tension of synthetic urine, i.e. 0.06 N/m.

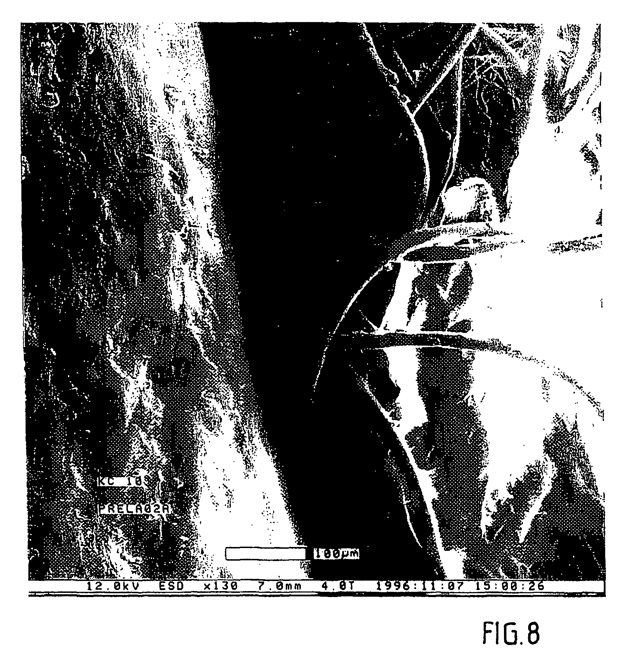

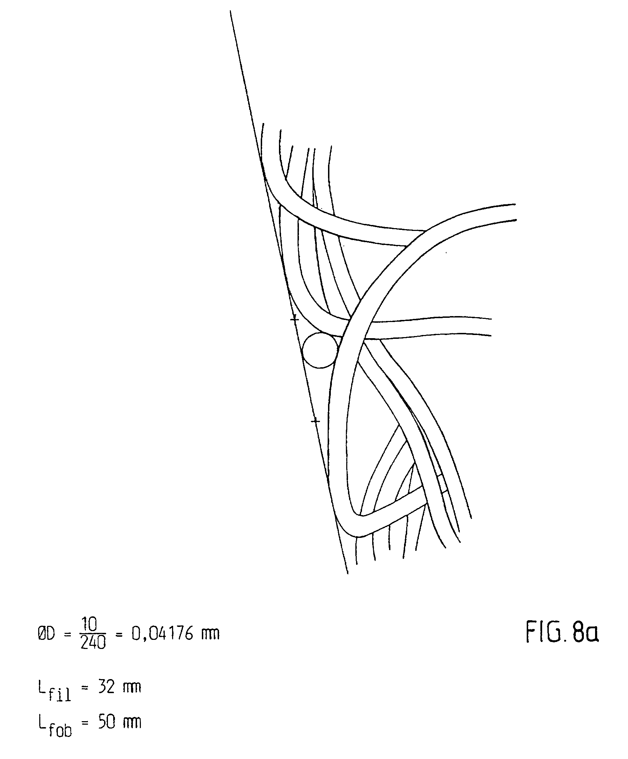

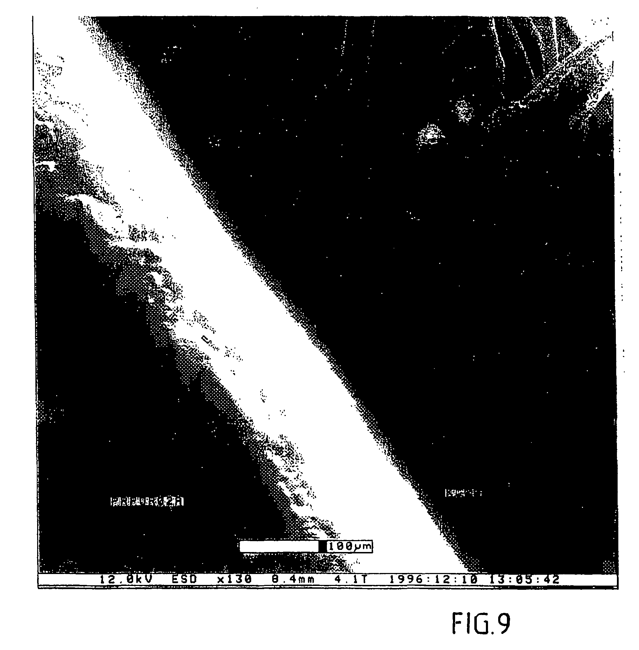

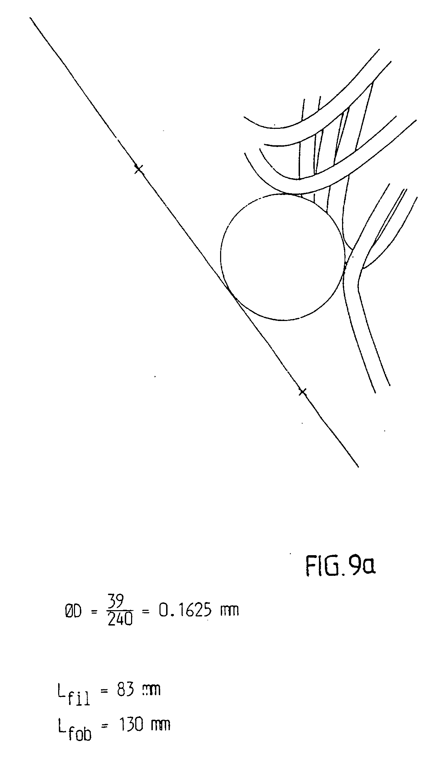

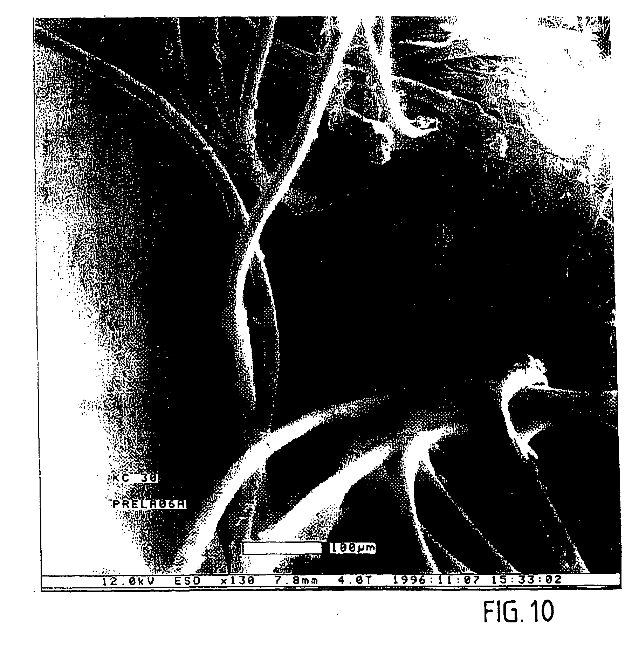

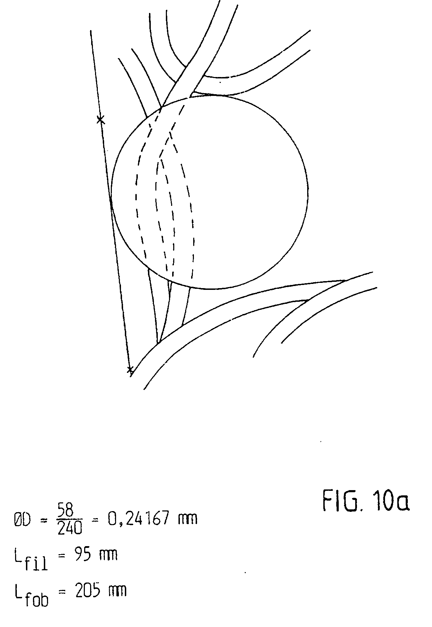

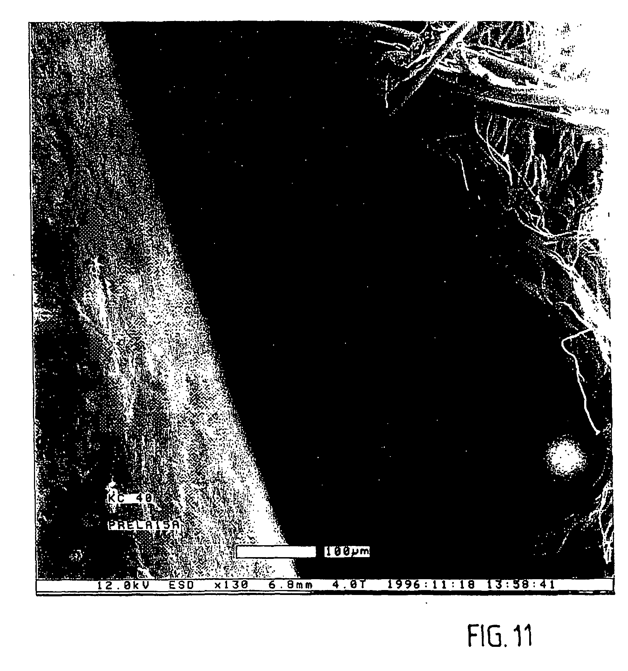

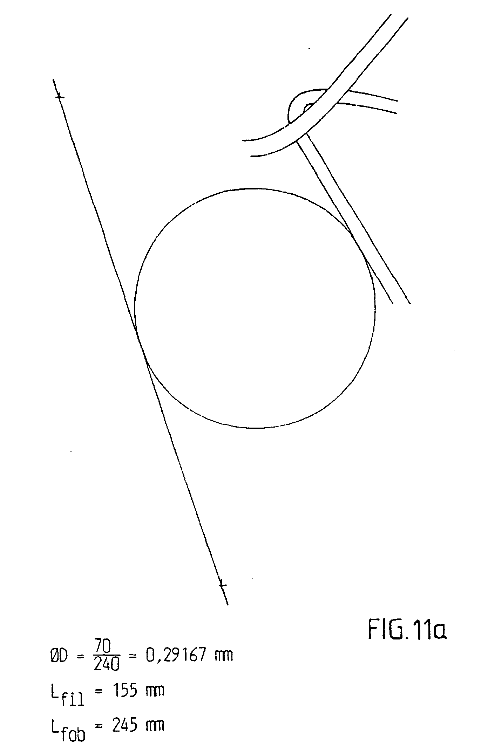

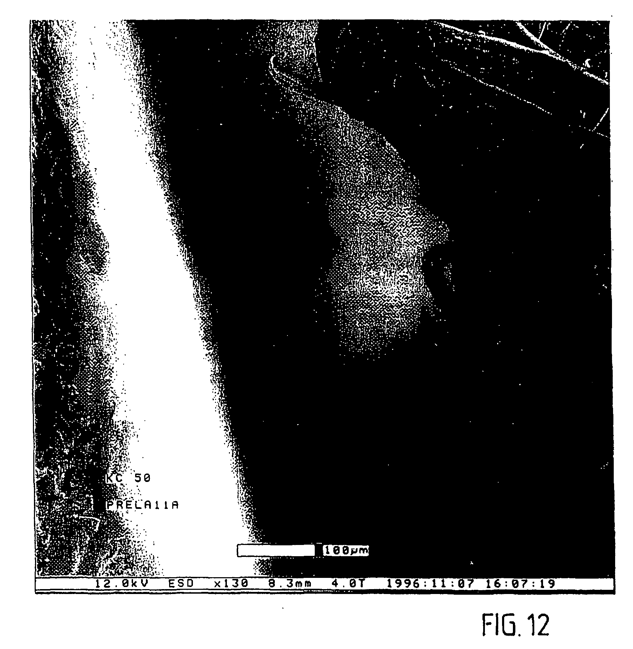

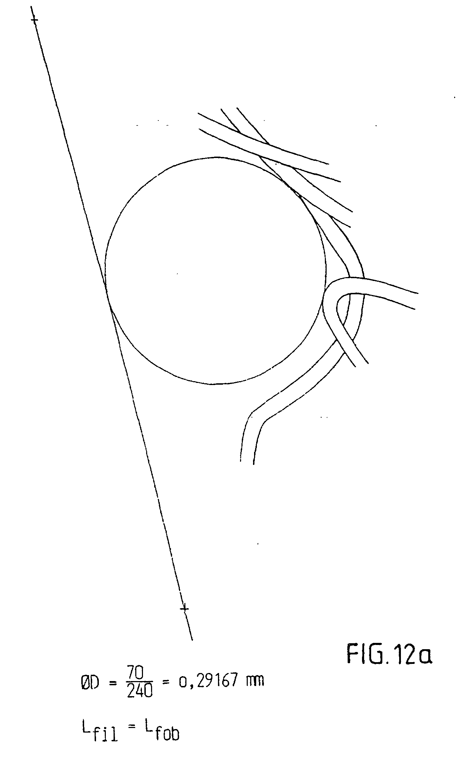

Abutment of a liquid barrier against the measuring equipment was studied at different available elongations with an electron microscope, enlargement 130 times, as illustrated in Figs. 8-12 and Figs. 8a-12a. As will be evident from the Figures, a through-penetrating pore is formed between the threads or fibres of the barrier material and the Plexiglas wall of the test equipment. This pore is assumed to function as a capillary, where r = the radius of the largest possible circle that can be enclosed in the channel, as evident from Fig. 2b.

The through-penetrating pore has been drawn in Figs. 8a-12a. The following pore-radius values were obtained at different available elongations, as shown in the Figures.

| **Available Elongation** | **Pore Radius** |
|---|---|
| 10% | 0.0208 mm |
| 20% | 0.0812 mm |
| 30% | 0.1208 mm |
| 40% | 0.1458 mm |
| 50% | 0.1458 mm |

Comments: It was very difficult to measure the pore radius on the photograph at a 10% available elongation, and the value given is therefore perhaps unreliable.

The crosses shown in Figs. 8a-12a show the lateral terminal points of the pore intended for calculating the hydrophobic and hydrophilic length-proportions of the pore. The length ratio between hydrophobic and hydrophilic surfaces in the pore at different available elongations is shown in the following Table.

| **Avail. elong** | **Hydrophil. surface** | **Hydrophob. surface** |
|---|---|---|
| 10% | 39% | 61% |
| 20% | 39% | 61% |
| 30% | 32% | 68% |
| 40% | 39% | 61% |
| 50% | 50% | 50% |

Calculations relating to "Huggies standing gather" against Plexiglas:

### 10% available elongation

ΔP = 2·0.06·(0.39·cos74°+0.61·cos120°)/0.0208·10⁻³
ΔP = -1139 Pa => the absolute value of the breakthrough pressure = 114 mm H₂O

### 20% available elongation

ΔP = 2·0.06·(0.39·cos74°+0.61·cos120°)/0.0812·10⁻³
ΔP = -291.9 Pa => the absolute value of the breakthrough pressure = 29.2 mm H₂O

### 30% available elongation

ΔP = 2·0.06·(0.32·cos74°+0.68·cos120°)/0.1208·10^{-3c}
ΔP = -250.1Pa => the absolute value of the breakthrough pressure = 25.0 mm H₂O

### 40% available elongation

ΔP = 2·0.06·(0.39·cos74°+0.61·cos120°)/0.1458·10⁻³
ΔP = -162.6 Pa => the absolute value of the breakthrough pressure = 16.3 mm H₂O

### 50% available elongation

ΔP = 2·0.06·(0.5·cos74°+0.5·cos120°)/0.1458·10⁻³
ΔP = -92.3 Pa => the absolute value of the breakthrough pressure = 9.2 mm H₂O

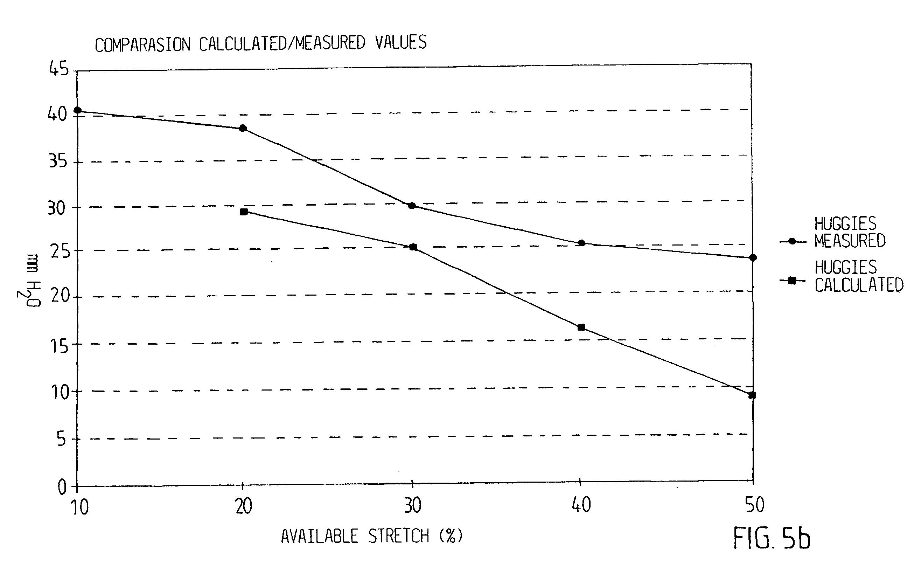

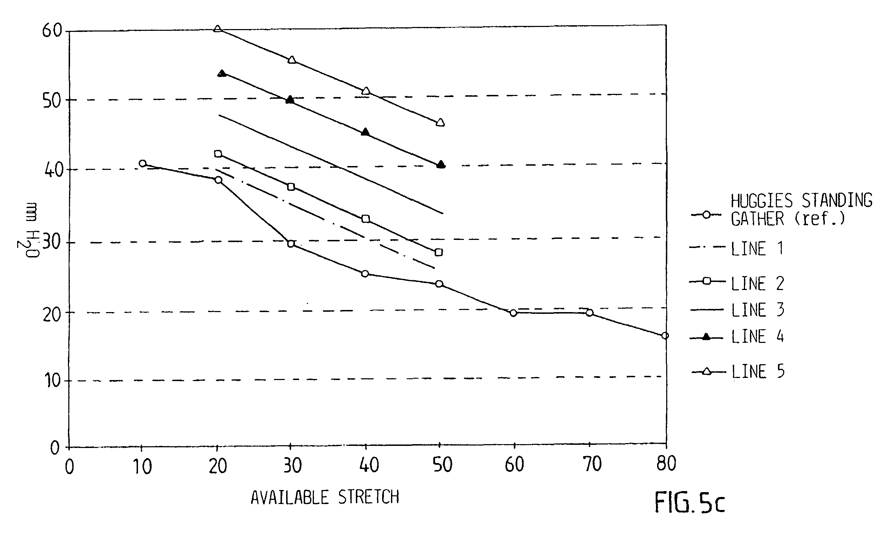

Fig. 5b shows a comparison between the breakthrough pressures measured with the test equipment and the breakthrough pressures calculated with the above formula.

Since the calculated and measured breakthrough pressures are in good agreement, it is thus possible to improve the sealing effect of the liquid barrier in an absorbent article against the wearer's skin by influencing |ΔP|, i.e. |(2γ cosθm/r)|, of the barrier so that this value increases. One provision in this respect is that the tension in the elastic will be sufficiently high to prevent the liquid barrier from allowing liquid to escape at a lower pressure as a result of the elastic relaxing and allowing the barrier to "ease" away from the wearer by virtue of the liquid column weighing down the barrier so that it releases its contact with the abutment surface.

|ΔP| can be caused to increase by increasing the product |(2γcosθm/r)|

The invention thus relates to a method of improving the sealing ability of at least one sealing edge in an absorbent article of the type described in the preamble to the specification by modifying or treating the absorbent article in such a way as to cause the absolute value of ΔP=2γ cosθm/r for said sealing edge (28, 29) to increase, where γ designates the surface tension of the liquid to be absorbed by suction, r designates the radius of the largest circle that can be encompassed in any pore formed by the sealing edge against the wearer's skin at the given available elongation, and cosθm is the weighted mean value of cosθ, where θ is the wetting angle of the liquid to the material in the pore walls, while taking into account the different materials in the walls of this largest pore.

The product can be increased by, for instance
1) influencing the wetting angle of the liquid to be sucked up to the skin or to the barrier material, respectively;
2) influencing the pore radius, i.e. the radius of the largest circle that can be encompressed in any pore formed between the barrier material and the skin; and
3) influencing both wetting angle and pore radius.

Because the effect intended is to increase the absolute value of the product 2γ cosθm/r, it is not necessary to unilaterally increase |cosθm| or decrease r. It is possible for a procedure of increasing |cosθm/r| to also involve simultaneous increase of the radius. Provided that the increase in |cosθm| is proportionally greater than the increase in radius, an improved result will be obtained despite the increase in radius. Similarly, a procedure that decreases the radius may result in a decrease in |cosθm|. An improved result will still be achieved, however, provided that this latter decrease is proportionally smaller than the decrease in radius.

The invention also relates to an absorbent article such as a diaper or an incontinence guard that has improved sealing properties against a user and which has been produced so that in the case of at least a pair of the liquid barriers of the article, the absolute value of the product 2γ cosθm/r will be higher than that obtained when using earlier known absorbent articles. More specifically, in respect of at least one sealing edge on each side of the centre line of said absorbent body, the absolute value of ΔP=2γ cosθm/r lies above the line y=kx+m, where x designates the available elongation, k has the value -14/30 and m has the value 48 (line 1), preferably 51 (line 2), more preferably 57 (line 3) and even more preferably 63 (line 4) and in particular 69 (line 5). These lines are shown in Fig. 5c, in which the measured breakthrough pressure of the most effective liquid barrier known at present, i.e. the Huggies standing gather barrier, has been drawn by way of comparison.

Preferably the absolute value of ΔP=2γ cosθm/r lies above the line y=kx+m during the major part of the interval between 15 and 50% available elongation or stretch, and particularly during the major part of the interval between 10 and 80% available elongation.

The invention will now be described in more detail with reference to particular exemplifying embodiments and also with reference to the accompanying drawings.

### Examples

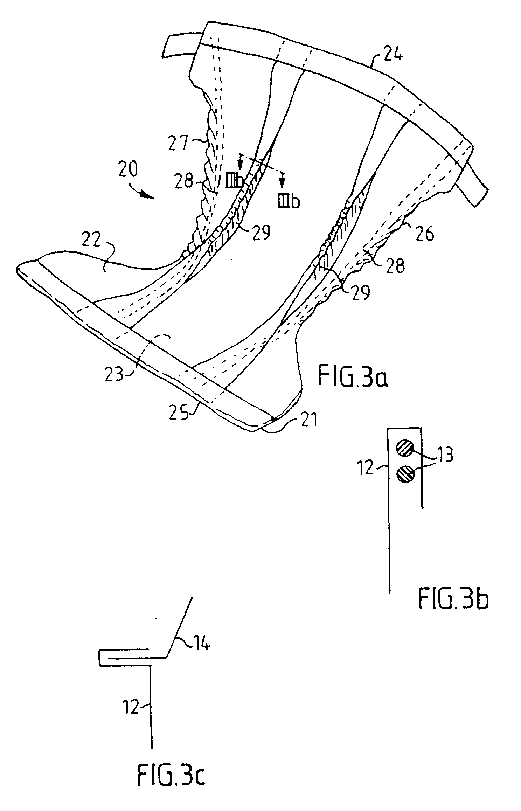

Fig. 3a shows a conventional diaper or incontinence guard 20 which includes a liquid-permeable upper sheet 22, an absorbent sheet 23, and a liquid-impermeable bottom sheet 21, said sheets being delimited by two transverse extremities 24, 25 and two longitudinal extremities 26, 27. The illustrated article also includes longitudinally extending leg elastic 28 and an upstanding liquid barrier 29 on each side of the longitudinal centre line. Fig. 3b is a sectional view that illustrates the construction of the upstanding liquid barrier comprising a liquid-impermeable sheet 12 whose free edge is curved around two stretched elastic threads 13. The threads 13 function to pucker the sheet 12.

Fig. 3c illustrates an embodiment of the invention in which the pore radius has been significantly reduced, and is constant and small already at high available elongation or stretch. In this case, the elastic threads 13 have been replaced by an elastic film 14. This film will lie essentially smoothly against the skin. The pore radius is close to 0 already at a high available elongation.

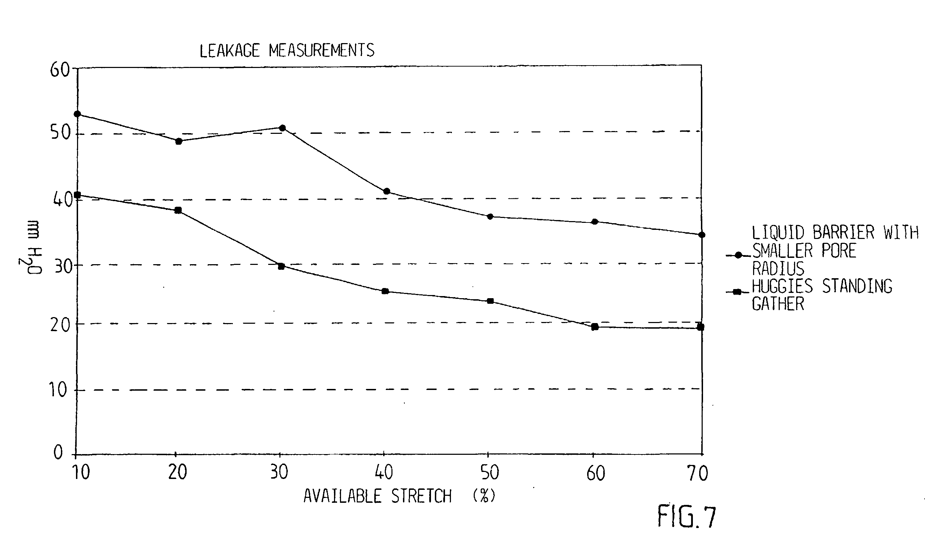

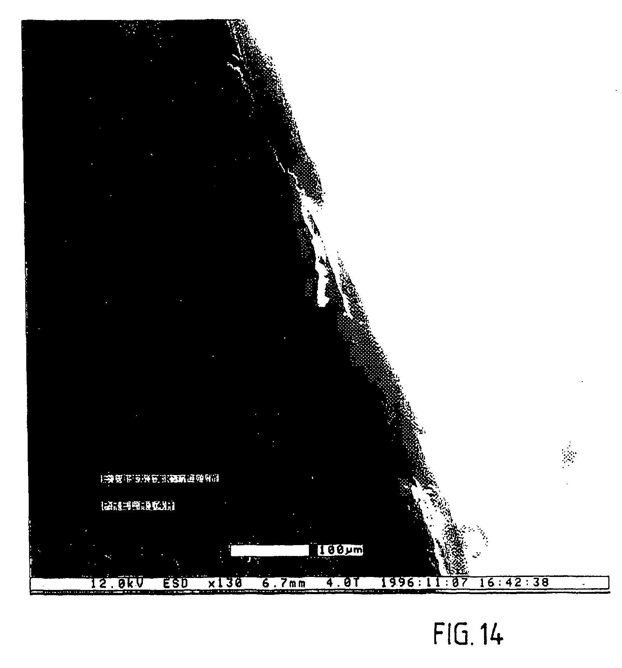

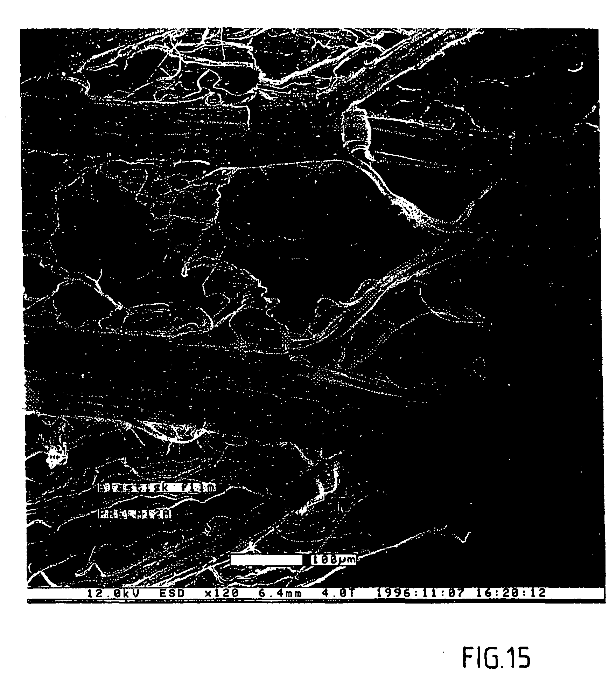

Fig. 14 is a reproduction of an electron microscope photograph of an inventive embodiment similar to the embodiment of Fig. 3c, in which an elastic film is used as the edge on the liquid barrier, stretched against the Plexiglas. The small pores that can be seen are the result of the film being a three-ply film in the illustrated case, where only the centre layer is elastic. The outer layers used to join the film to the barrier will crack when the centre layer is stretched, as shown in Fig. 15. The visible small pores, which have a radius of about 0.02 mm, will remain essentially constant irrespective of the available elongation, since the height of the pores is constant even though the length is changed. The uppermost curve in Fig. 7 illustrates measurements made with this liquid barrier. The lower curve shows the results obtained with a Huggies standing gather.

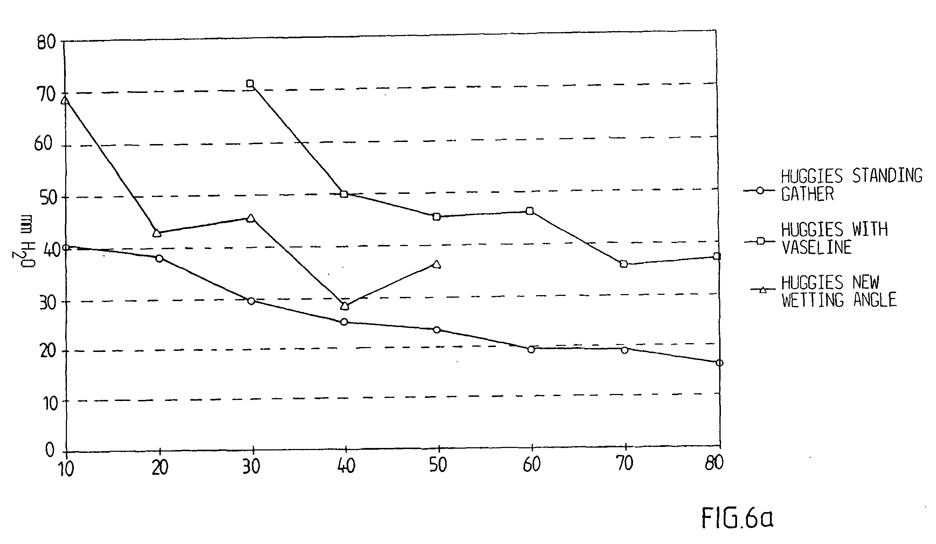

The wetting angle was changed in two tests. In the first case, a plastic film against which the liquid has a wetting angle of 97.5° was stretched over the first semi-cylindrical Plexiglas surface. This corresponds to a treatment of the barrier such that the liquid will obtain a higher wetting angle to the wearer's skin. This is hydrophobic in distinction to the normal mean wetting angle of the liquid to skin of about 74°. The result of this change in the wetting angle (centre curve) is compared in Fig. 6a with the sealing effect achieved with the upstanding liquid barrier Huggies standing gather (lowermost curve). As the measuring values show, an improved sealing effect is achieved in this way.

The uppermost curve in Fig. 6a shows measurements obtained with a liquid barrier that had been treated with Vaseline. The liquid has a wetting angle of 100° to Vaseline The Vaseline partially blocks the pores, i.e. reduces the pore radius, and also smears the wearer's skin, thereby increasing the wetting angle of the liquid to the skin. As will be evident from the diagram shown in Fig. 6a, there is obtained a significant improvement that exceeds the improvement achieved when only the wetting angle of the liquid to the skin is changed, despite obtaining, at the same time, a reduction in the wetting angle of the liquid to the barrier by virtue of the Vaseline also smearing the liquid barrier and therewith lowering the wetting angle from 120° to 100°.

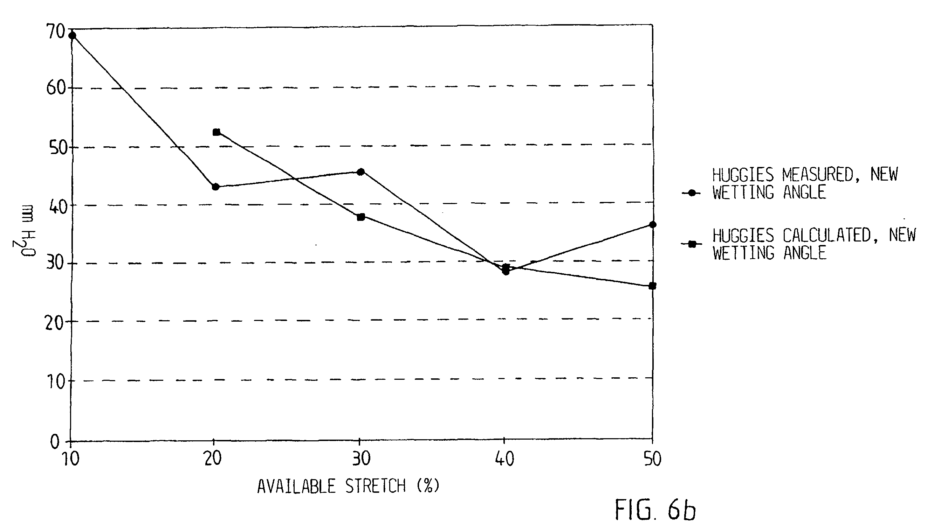

Fig. 6b is a diagram in which the calculated and measured sealing effects obtained when changing the wetting angle are shown. The measured values have been obtained by covering the Plexiglas with the aforedescribed plastic film, and corresponds to the centre curve in the diagram shown in Fig. 6a. Good-agreement is obtained between the calculated and measured values.

As will be evident from the aforegoing, the sealing effect provided by an absorbent article can be improved by treating at least one of its liquid barriers in a manner such as to cause the absolute value of ΔP=2γ cosθm/r to increase at least within the major part of an available elongation range of 20-40%. The sealing effect of an article having a liquid barrier where the absolute value of ΔP=2γ cosθm/r lies above the line y=kx+m at least within the major part of an available elongation range of 20-40%, where x designates the available elongation or stretch, k has the value -14/30 and m has the value 48, preferably 51, more preferably 57, and even more preferably 63 and particularly 69, will be substantially better than the sealing effect achieved with conventional articles of this nature.

The invention also relates to articles that have transverse liquid barriers, and to a method of treating such liquid barriers in the same manner as that described with respect to the longitudinally extending liquid barriers.

The longitudinally extending liquid barriers may be comprised of both leg elastic and inner cuffs.

It will be understood that the invention is not restricted to the described exemplifying embodiments thereof and that it includes all conceivable embodiments that lie within the scope of the following Claims.

## Claims

1. A method of achieving, in an absorbent article, such as a diaper or an incontinence guard, that includes an absorbent body (23) disposed between a liquid-impermeable bottom sheet (21) which is intended to lie distal from the wearer in use, a liquid-permeable upper sheet (22) which is intended to lie proximal to the wearer, and either 1) at least one longitudinally extending liquid barrier (28, 29) on each side of the centre line of said upper sheet, made of essentially liquid-impervious material (12) and fastened along or adjacent to a respective longitudinally extending side extremity of the article and comprising a free elastic sealing edge intended to be stretched against the wearer, or 2) above the upper sheet (22), a top liquid-impermeable sheet which is intended to lie against the wearer, includes elastic for shaping the article to the wearer's body, and includes apertures intended to lie in register with the anus and the urethra orifice of the wearer, around which apertures elastically puckered sealing edges, are disposed in the top sheet,
an improved sealing ability against the skin of an intended wearer, at a given available elongation, by at least one sealing edge (28, 29) on each side of said centre line, **characterized by** modifying or treating the absorbent article in such a way as to cause the absolute value of ΔP=2γ cosθm/r for said sealing edge (28, 29) to increase, where γ designates the surface tension of the liquid to be absorbed by suction, r designates the radius of the largest circle that can be encompassed in any pore formed by the sealing edge against the wearer's skin at the given available elongation, and cosθm is the weighted mean value of cosθ, where θ is the wetting angle of the liquid to the material in the pore walls, while taking into account the different materials in the walls of this largest pore.

2. A method according to Claim 1, **characterized by** causing the absolute value of ΔP to increase at least within the major part of an available elongation range of 20-40%.

3. A method according to Claim 1 or 2, **characterized by** causing the absolute value of ΔP to increase by at least 5%, particularly by at least 15%, preferably by at least 25%, and then particularly by at least 35%.

4. A method according to any one of the preceding Claims, **characterized by** causing the pore radius of the sealing edge to decrease at least at an available elongation above 60%, particularly at an available elongation above 50%, more particularly at an available elongation above 40% and then preferably at an available elongation above 20%.

5. A method according to any one of the preceding Claims, **characterized by** causing the absolute value of cosθm to increase.

6. A method according to Claim 5, **characterized by** treating the sealing edge such that a higher wetting angle of the liquid to the barrier material will be obtained and/or such that a higher wetting angle of the liquid to the skin of the wearer will be obtained within those regions in which the sealing edge lies against the skin when the absorbent article is donned.

7. A method according to any one of the preceding Claims, **characterized by** providing the sealing edge with a layer of material that increases the absolute value of cosθm and/or that reduces r when the article is donned.

8. A method according to any one of Claims 1-7, **characterized by** causing the absolute value of cosθm/r to increase.

9. An absorbent article that includes an absorbent body (23) disposed between a liquid-impermeable bottom sheet (21) which is intended to lie distal from the wearer in use, a liquid-permeable upper sheet (22) which is intended to lie proximal to the wearer, and either 1) at least one longitudinally extending liquid barrier (28, 29) on each side of the centre line of said upper sheet, made of essentially liquid-impervious material (12) and fastened along or adjacent to a respective longitudinally extending side extremity of the article and comprising a free elastic sealing edge intended to be stretched against the wearer, or 2) above the upper sheet (22), a liquid-impermeable top sheet which is intended to lie against the wearer, includes elastic for shaping the article to the wearer's body, and includes apertures intended to lie in register with the anus and the urethra orifice of the wearer, around which apertures elastically puckered sealing edges are disposed in the top sheet, **characterized in that** in respect of at least one sealing edge (28, 29) on each side of the centre line of said absorbent body the absolute value of ΔP=2γ cosθm/r lies above the line y=kx+m, where x designates the available elongation, k has the value -14/30 and m has the value 48, particularly 51, preferably 57, more preferably 63 and in particular 69, within the major part of an available elongation range of between 20 and 40% and where γ designates the surface tension of the liquid to be absorbed, r designates the radius of the largest circle that can be enclosed in any pore formed by the sealing edge against the skin of the wearer at a given available elongation, and cosθm is the weighted mean value of cosθ, where θ is the wetting angle of the liquid to the material in the pore walls while taking into account the different materials in the walls of the largest pore.

10. An article according to Claim 9, **characterized in that** the free sealing edge includes a layer of a material such that a higher wetting angle of the liquid to the edge material will be obtained and/or such that a higher wetting angle of the liquid to the skin of the wearer will be obtained within those regions in which the sealing edge lies against the skin and where said material smears the skin when the absorbent article is donned.

11. An article according to Claim 9 or 10, **characterized in that** the free sealing edge is provided with a layer of a material which at least partly fills out the pores in said edge when the article is donned.

12. An article according to any one of Claims 9-11, **characterized in that** when the article is donned, the free sealing edge has a pore radius which is essentially independent of the available elongation or stretch and which is at most 0.10 mm, preferably at most 0.08 mm and most preferably at most 0.04 mm.

13. An article according to any one of Claims 9-12, **characterized in that** the free sealing edge is comprised of a ribbon-like elastic film.

14. An article according to any one of Claims 9-13, **characterized in that** the absolute value of ΔP=2γ cosθm/r lies above the line y=kx+m within the major part of an available elongation range of 15-50%, preferably within the major part of the range 10-60%.

## Patentansprüche

1. Verfahren zum Erlangen in einem Absorptionsartikel, wie z.B. einer Windel oder einem Inkontinenzschutz, der einen Absorptionskörper (23) aufweist, der zwischen einer flüssigkeitsundurchlässigen Unterlage (21), die dafür vorgesehen ist, während der Verwendung distal von dem Benutzer zu liegen und einer flüssigkeitsdurchlässigen Oberlage (22), die dafür vorgesehen ist, proximal zu dem Benutzer zu liegen angeordnet ist, und entweder 1) wenigstens eine sich in Längsrichtung erstreckende Flüssigkeitsbarriere (28, 29) an jeder Seite der Mittellinie der Oberlage, die aus im Wesentlichen flüssigkeitsundurchlässigen Material (12) ausgeführt ist, und entlang oder benachbart zu einer jeweiligen, sich in Längsrichtung erstreckenden Seitenextremität des Artikels befestigt ist und einen freien elastischen Dichtrand aufweist, der dafür .vorgesehen ist, gegen den Benutzer gedehnt zu werden, oder 2) oberhalb der Oberlage (22), eine obere flüssigkeitsundurchlässige Lage, die dafür vorgesehen ist, gegen den Benutzer zu liegen, eine Elastik zum Formen des Artikels an den Körper des Benutzers aufweist und Aussparungen aufweist, die dafür vorgesehen sind, mit dem Anus und der Harnröhrenöffnung des Benutzers ausgerichtet zu liegen, um welche Aussparungen elastisch gefältelte Dichtränder in der oberen Lage angeordnet sind, aufweist,
einer verbesserten Dichtfähigkeit gegen die Haut eines beabsichtigten Benutzers bei einer gegebenen verfügbaren Verlängerung, durch wenigstens einen Dichtrand (28, 29) an jeder Seite der Mittellinie,
**gekennzeichnet durch** Modifizieren oder Behandeln des Absorptionsartikels auf derartige Art und Weise, dass der Absolutwert von ΔP=2γcosθm/r für den Dichtrand (28, 29) veranlasst wird, sich zu vergrößern, wobei γ die Oberflächenspannung der **durch** Saugen zu absorbierenden Flüssigkeit bezeichnet, r den Radius des größten Kreises bezeichnet, der in eine beliebige Pore eingeschrieben werden kann, die **durch** den Dichtrand gegen die Haut des Benutzers bei der gegebenen verfügbaren Verlängerung ausgebildet ist, und cosθm der gewichtete Durchschnittswert von cosθ ist, wobei θ der Nässwinkel der Flüssigkeit zu dem Material in den Porenwänden ist, während die unterschiedlichen Materialien in den Wänden dieser größten Pore in Betracht gezogen werden.

2. Verfahren nach Anspruch 1, **gekennzeichnet durch** Veranlassen des Absolutwerts von ΔP, sich wenigstens innerhalb des wesentlichen Teils des verfügbaren Verlängerungsbereichs von 20-40% zu vergrößern.

3. Verfahren nach Anspruch 1 oder 2, **gekennzeichnet durch** Veranlassen des Absolutwerts ΔP, sich um wenigstens 5%, insbesondere wenigstens 15%, vorzugsweise wenigstens 25%, und dann insbesondere wenigstens 35% zu vergrößern.

4. Verfahren nach einem der vorangehenden Ansprüche, **gekennzeichnet durch** Veranlassen des Porenradius des Dichtrandes, sich wenigstens bei einer verfügbaren Verlängerung oberhalb 60% zu verringern, insbesondere bei einer verfügbaren Verlängerung oberhalb 50%, insbesondere bei einer verfügbaren Verlängerung oberhalb 40% und dann vorzugsweise bei einer verfügbaren Verlängerung oberhalb 20%.

5. Verfahren nach einem der vorangehenden Ansprüche, **gekennzeichnet durch** Veranlassen des Absolutwerts von cosθm, sich zu erhöhen.

6. Verfahren nach Anspruch 5, **gekennzeichnet durch** Behandeln des Dichtrandes derart, dass ein höherer Nässwinkel der Flüssigkeit zu dem Barrierenmaterial erreicht wird, und/oder derart, dass ein höherer Nässwinkel der Flüssigkeit zu der Haut des Benutzers innerhalb derjenigen Bereiche erhalten wird, in denen der Dichtrand gegen die Haut liegt, wenn der Absorptionsartikel getragen wird.

7. Verfahren nach einem der vorangehenden Ansprüche, **gekennzeichnet durch** Versehen des Dichtrandes mit einer Materialschicht, welche den Absolutwert von cosθm erhöht und/oder r verringert, wenn der Artikel getragen wird.

8. Verfahren nach einem der Ansprüche 1 bis 7, **gekennzeichnet durch** Veranlassen des Absolutwerts von cosθm/r, sich zu erhöhen.

9. Absorptionsartikel mit einem Absorptionskörper .(23), der zwischen einer flüssigkeitsundurchlässigen Unterlage (21), die dafür vorgesehen ist, während der Verwendung distal von dem Benutzer zu liegen und einer flüssigkeitsundurchlässigen Oberlage (22), die dafür vorgesehen ist, proximal zu dem Benutzer zu liegen angeordnet ist, und entweder 1) wenigstens einer sich in Längsrichtung erstreckenden Flüssigkeitsbarriere (28, 29) an jeder Seite der Mittellinie der Oberlage, die aus im Wesentlichen flüssigkeitsundurchlässigen Material (12) ausgeführt ist, und'entlang oder benachbart an eine jeweilige, sich in Längsrichtung erstreckende Seitenextremität des Artikels befestigt ist und einen freien elastischen Dichtrand aufweist, der dafür vorgesehen ist, gegen den Benutzer gedehnt zu werden, oder 2) oberhalb der Oberlage (22) einer flüssigkeitsundurchlässigen oberen Lage, die dafür vorgesehen ist, gegen den Benutzer zu liegen, eine Elastik zum Formen des Artikels an den Körper des Benutzers aufweist, und Aussparungen aufweist, die dafür vorgesehen sind, mit dem Anus und der Harnröhrenöffnung des Benutzers ausgerichtet zu liegen, um welche Aussparungen elastisch gefältelte Dichtränder in der oberen Lage angeordnet sind,
**dadurch gekennzeichnet, dass** bezüglich wenigstens eines Dichtrandes (28, 29) an jeder Seite der Mittellinie des Absorptionskörpers der Absolutwert von ΔP=2γcosθm/r oberhalb der Linie y=kx+m liegt, wobei x die verfügbare Verlängerung bezeichnet, k den Wert -14/30 aufweist, und m den Wert 48 aufweist, insbesondere 51, vorzugsweise 57, weiter bevorzugt 63 und insbesondere 69, und zwar innerhalb des Hauptteils eines verfügbaren Verlängerungsbereichs von zwischen 20 und 40%, und wobei γ die Oberflächenspannung der zu absorbierenden Flüssigkeit bezeichnet, r den Radius des größten Kreises bezeichnet, der in eine Pore eingeschlossen werden kann, die durch den Dichtrand gegen die Haut des Benutzers bei einer gegebenen verfügbaren Verlängerung ausgebildet ist, und cosθm der gewichtete Durchschnittswert von cosθ ist, wobei θ der Nässwinkel der Flüssigkeit zu dem Material in den Porenwänden ist, während die unterschiedlichen Materialien in den Wänden der größten Pore in Betracht gezogen werden.

10. Artikel nach Anspruch 9, **dadurch gekennzeichnet, dass** der freie Dichtrand eine Materialschicht derart aufweist, dass ein höherer Nässwinkel der Flüssigkeit zu dem Randmaterial erhalten wird, und/oder ein derartiger höherer Nässwinkel der Flüssigkeit zu der Haut des Benutzers innerhalb derjenigen Bereiche erhalten wird, in denen der Dichtrand gegen die Haut liegt, und wo das Material die Haut schmiert, wenn der Absorptionsartikel getragen wird.

11. Artikel nach Anspruch 9 oder 10, **dadurch gekennzeichnet, dass** der freie Dichtrand mit einer Materialschicht versehen ist, die wenigstens teilweise die Poren in dem Rand ausfüllt, wenn der Artikel getragen wird.

12. Artikel nach einem der Ansprüche 9 bis 11, **dadurch gekennzeichnet, dass**, wenn der Artikel getragen wird, der freie Dichtrand einen Porenradius aufweist, der im Wesentlichen unabhängig von der verfügbaren Verlängerung oder Dehnung ist, und der höchstens 0,10 mm, vorzugsweise höchstens 0,08 mm und besonders bevorzugt höchstens 0,04 mm beträgt.

13. Artikel nach einem der Ansprüche 9 bis 12, **dadurch gekennzeichnet, dass** der freie Dichtrand aus einer bandartigen, elastischen Folie besteht.

14. Artikel nach einem der Ansprüche 9 bis 13, **dadurch gekennzeichnet, dass** der Absolutwert von ΔP=2γcosθm/r oberhalb der Linie y=kx+m liegt, und zwar innerhalb des Hauptteils eines verfügbaren Verlängerungsbereichs von 15-50%, vorzugsweise innerhalb des Hauptteils des Bereichs von 10-60%.

## Revendications

1. Procédé d'obtention, dans un article absorbant, tel qu'une couche-culotte ou une protection contre l'incontinence, qui comprend un corps absorbant (23) placé entre une feuille inférieure imperméable aux liquides (21) qui, en utilisation, est destinée à se trouver en position distale par rapport à l'utilisateur, une feuille supérieure perméable aux liquides (22) qui est destinée à se trouver en position proximale par rapport à l'utilisateur, et soit 1) au moins une barrière pour liquide s'étendant longitudinalement (28, 29) de chaque côté de l'axe de ladite feuille supérieure, constituée d'un matériau essentiellement imperméable aux liquides (12) et fixée le long de ou adjacente à une extrémité latérale respective s'étendant longitudinalement de l'article et comprenant un bord d'étanchéité élastique libre destiné à être étiré sur l'utilisateur, soit 2) au-dessus de la feuille supérieure (22), une feuille de sommet imperméable aux liquides qui est destinée à se trouver contre l'utilisateur, comprend un élastique pour que l'article épouse la forme du corps de l'utilisateur, et comprend des ouvertures destinées à se trouver en coïncidence avec l'anus et le méat urinaire de l'utilisateur, autour desquelles sont placés dans la feuille de sommet des bords d'étanchéité froncés de façon élastique,
d'une capacité d'étanchéité améliorée contre la peau d'un utilisateur, pour un allongement disponible donné, au moyen d'au moins un bord d'étanchéité (28, 29) de chaque côté dudit axe, **caractérisé par** le fait de modifier ou de traiter l'article absorbant de manière à faire augmenter la valeur absolue de ΔP=2γcosθm/r pour ledit bord d'étanchéité (28, 29), γ désignant la tension superficielle du liquide à absorber par succion, r désignant le rayon du plus grand cercle qui peut être inscrit dans n'importe quel pore formé par le bord d'étanchéité contre la peau de l'utilisateur à l'allongement disponible donné, et cosθm étant la valeur moyenne pondérée de cosθ, θ étant l'angle de mouillage du liquide au matériau dans les parois de pore, en tenant compte des différents matériaux présents dans les parois de ce pore le plus grand.

2. Procédé selon la revendication 1, **caractérisé par** le fait de faire augmenter la valeur absolue de ΔP au moins dans la majeure partie d'une plage d'allongement disponible de 20-40 %.

3. Procédé selon la revendication 1 ou 2, **caractérisé par** le fait de faire augmenter la valeur absolue de ΔP d'au moins 5 %, en particulier d'au moins 15 %, de préférence d'au moins 25 %, et mieux encore d'au moins 35 %.

4. Procédé selon l'une quelconque des revendications précédentes, **caractérisé par** le fait de faire diminuer le rayon de pore du bord d'étanchéité au moins à un allongement disponible supérieur à 60 %, en particulier à un allongement disponible supérieur à 50 %, plus particulièrement à un allongement disponible supérieur à 40 %, et mieux encore à un allongement disponible supérieur à 20 %.

5. Procédé selon l'une quelconque des revendications précédentes, **caractérisé par** le fait de faire, augmenter la valeur absolue de cosθm.

6. Procédé selon la revendication 5, **caractérisé par** le fait de traiter le bord d'étanchéité de façon à obtenir un plus grand angle de mouillage du liquide au matériau de la barrière et/ou de façon à obtenir un plus grand angle de mouillage du liquide à la peau de l'utilisateur dans les régions où le bord d'étanchéité se trouve contre la peau lorsque l'utilisateur a revêtu l'article absorbant.

7. Procédé selon l'une quelconque des revendications précédentes, **caractérisé par** le fait de munir le bord d'étanchéité d'une couche de matériau qui augmente la valeur absolue de cosθm et/ou diminue r lorsque l'utilisateur a revêtu l'article absorbant.

8. Procédé selon l'une quelconque des revendications 1 à 7, **caractérisé par** le fait de faire augmenter la valeur absolue de cosθm/r.

9. Article absorbant qui comprend un corps absorbant (23) placé entre une feuille inférieure imperméable aux liquides (21) qui, en utilisation, est destinée à se trouver en position distale par rapport à l'utilisateur, une feuille supérieure perméable aux liquides (22) qui est destinée à se trouver en position proximale par rapport à l'utilisateur, et soit 1) au moins une barrière pour liquide s'étendant longitudinalement (28, 29) de chaque côté de l'axe de ladite feuille supérieure, constituée d'un matériau essentiellement imperméable aux liquides (12) et fixée le long de ou adjacente à une extrémité latérale respective s'étendant longitudinalement de l'article et comprenant un bord d'étanchéité élastique libre destiné à être étiré sur l'utilisateur, soit 2) au-dessus de la feuille supérieure (22), une feuille de sommet imperméable aux liquides qui est destinée à se trouver contre l'utilisateur, comprend un élastique pour que l'article épouse la forme du corps de l'utilisateur, et comprend des ouvertures destinées à se trouver en coïncidence avec l'anus et le méat urinaire de l'utilisateur, autour desquelles sont placés dans la feuille de sommet des bords d'étanchéité froncés de façon élastique, **caractérisé en ce que**, concernant au moins un bord d'étanchéité (28, 29) de chaque côté de l'axe dudit corps absorbant, la valeur absolue de ΔP=2γcosθm/r se trouve au-dessus de la droite d'équation y=kx+m, x désignant l'allongement disponible, k étant égal à -14/30 et m étant égal à 48, en particulier 51, de préférence 57, mieux encore 63 et plus particulièrement 69, dans la majeure partie d'une plage d'allongement disponible comprise entre 20 et 40 %, et γ désignant la tension superficielle du liquide à absorber, r désignant le rayon du plus grand cercle qui peut être inscrit'dans n'importe quel pore formé par le bord d'étanchéité contre la peau de l'utilisateur à un allongement disponible donné, et cosθm étant la valeur moyenne pondérée de cosθ, θ étant l'angle de mouillage du liquide au matériau dans les parois de pore, en tenant compte des différents matériaux présents dans les parois du pore le plus grand.

10. Procédé selon la revendication 9, **caractérisé en ce que** le bord d'étanchéité libre comprend une couche de matériau qui permet d'obtenir un plus grand angle de mouillage du liquide au matériau du bord et/ou d'obtenir un plus grand angle de mouillage du liquide à la peau de l'utilisateur dans les régions où le bord d'étanchéité se trouve contre la peau et où ledit matériau s'étale sur la peau lorsque l'utilisateur a revêtu l'article absorbant.

11. Article selon la revendication 9 ou 10, **caractérisé en ce que** le bord d'étanchéité libre est muni d'une couche de matériau qui remplit au moins partiellement les pores dans ledit bord lorsque l'utilisateur a revêtu l'article absorbant.

12. Article selon l'une quelconque des revendications 9 à 11, **caractérisé en ce que** lorsque l'article est porté, le bord d'étanchéité libre a un rayon de pore qui est essentiellement indépendant de l'allongement ou étirement disponible et qui vaut au plus 0,10 mm, de préférence au plus 0,08 mm, et mieux encore au plus 0,04 mm.

13. Article selon l'une quelconque des revendications 9 à 12, **caractérisé en ce que** le bord d'étanchéité libre est constitué d'un film élastique en forme de ruban.

14. Article selon l'une quelconque des revendications 9 à 13, **caractérisé en ce que** la valeur absolue de ΔP=2γcosθm/r se trouve au-dessus de la droite d'équation y=kx+m dans la majeure partie d'une plage d'allongement disponible de 15-50 %, de préférence dans la majeure partie de la plage de 10-60 %.
